# EUROPÄISCHE PATENTSCHRIFT

(11) **EP 0 328 883 B1**
(45) Veröffentlichungstag und Bekanntmachung des Hinweises auf die Patenterteilung: **06.07.1994**
(21) Anmeldenummer: 89100706.4
(22) Anmeldetag: 17.01.1989
(51) Int. Cl.: A61B 17/58, A61F 5/02

(54) **Stützvorrichtung für die menschliche Wirbelsäule**
Supporting device for the human spinal column
Dispositif d'étaiement de la colonne vertébrale humaine

(30) Priorität: 18.02.1988 DE 8802112 U
(43) Veröffentlichungstag der Anmeldung: 23.08.1989
(73) Patentinhaber: Howmedica GmbH, D-24232 Schönkirchen (DE)
(72) Erfinder: Metz-Stavenhagen,Peter,Dr.med., D-3590 Bad Wildungen (DE); Behrens,Klaus Friedrich Adolf, D-2351 Rickling (DE)
(74) Vertreter: Patentanwälte Hauck, Graalfs, Wehnert, Döring, Siemons

(56) Entgegenhaltungen:
- EP-A- 0 140 786
- DE-A- 3 132 520
- DE-A- 3 219 575
- DE-A- 3 306 657
- DE-U- 8 711 317
- DE-U- 8 712 943
- FR-A- 2 289 164
- FR-A- 2 506 606
- GB-A- 2 051 581
- GB-A- 2 131 300
- GB-A- 2 178 323
- US-A- 1 997 466
- US-A- 4 382 438
- US-A- 4 611 580

## Beschreibung

Die Neuerung bezieht sich auf eine Stützvorrichtung für die menschliche Wirbelsäule nach dem Oberbegriff des Anspruchs 1.

Aus dem DE-Gm 87 11 317 ist eine Stützvorrichtung für die Wirbel der menschlichen Wirbelsäule bekanntgeworden, bei der sogenannte Schanz'sche Schrauben über die Pedikel in den Wirbelkörper eingeschraubt werden. Sie sind in Haltebuchsen aufgenommen, die drehbar in Haltekörpern gelagert sind. Die Haltekörper sind ihrerseits auf das Ende eines Gewindebolzens aufschraubbar. Die bekannte Stützvorrichtung ermöglicht eine Verstellung der Schanz'schen Schrauben bezüglich drei Freiheitsgrade; sie kann daher nicht nur im richtigen Winkel in den Bogenfuß eingeschraubt werden, sondern ermöglicht auch anschließend eine frei im Raum liegende Bewegung, um zum Beispiel zwei Wirbel relativ zueinander in gewünschter Weise zu verstellen. Dies ist zum Beispiel nötig, um eine Reposition von Wirbeln durchzuführen und/oder einen frakturierten Wirbel von den benachbarten Wirbeln zu entlasten. Die bekannte Stützvorrichtung ist indessen nicht in der Lage, mehr als einen Wirbel zu überbrücken oder etwa im Fall von sogenannten Etagenbrüchen eine multisegmentäre Versorgung zu gewährleisten.

Es ist auch bereits bekanntgeworden, Pedikelschrauben an einem Gewindedraht festzulegen. Die Pedikelschrauben weisen einen Gabelkopf auf, der zwei durch einen Steg beabstandete zylindrische zur Gabelöffnung hin geöffnete Bohrungsabschnitte aufweist, in die Zylinderabschnitte von Muttern einführbar sind, die auf den Gewindedraht geschraubt sind. Mit Hilfe von zwei Muttern kann eine Pedikelschraube sowohl in der Achslage auf dem Draht als auch in ihrer Drehlage auf dem Gewindedraht festgelegt werden. Mit Hilfe einer derartigen Stützvorrichtung, bei der der Gewindedraht dorsal implantiert wird, können Pedikelschrauben in einer Reihe von Wirbeln gesetzt werden. Die bekannte Stützvorrichtung ermöglicht jedoch wegen ungenügender Festigkeit keine primär stabile Abstützung. Sie kann daher im wesentlichen nur verwendet werden für in sich stabile Wirbelsäulen, deren Verlauf aus orthopädischen Gründen korrigiert werden soll. Für eine Versorgung von Wirbelsäulenfrakturen, insbesondere bei den Mehretagenbrüchen, ist die bekannte Vorrichtung nicht geeignet.

Der Erfindung liegt daher die Aufgabe zugrunde, eine Stützvorrichtung für die menschliche Wirbelsäule zu schaffen, mit der mehr als ein Wirbel überbrückt werden kann und die eine primäre Stabilisierung der Wirbel bezüglich aller Freiheitsgrade ermöglicht.

Diese Aufgabe wird erfindungsgemäß durch die Merkmale des des Anspruchs 1 gelöst.

Die erfindungsgemäße Vorrichtung weist mindestens zwei getrennte, vorzugsweise mit Gewinde versehene Bolzen auf, die mit einer vorzugsweise ein Gewinde aufweisenden Hülse zusammenwirken. Die Kopfenden der Bolzen sind mit einer Klemmfläche versehen, die koaxial oder achsparallel angeordnet ist. Mit Hilfe einer geeigneten Befestigungsvorrichtung können beliebig viele, durch die Hülse verbundene Bolzenpaare miteinander verbunden werden, wobei die Klemmfläche eines Bolzenpaares mit einer Klemmfläche des benachbarten Bolzenpaares in klemmenden Eingriff gebracht wird. Die Pedikelschrauben haben ihrerseits Klemmflächen und werden mit Hilfe einer Befestigungsvorrichtung an einer Klemmfläche eines Bolzenkopfes festgelegt bzw. zwischen den Klemmflächen miteinander verbundener Bolzenköpfe. Die Enden eines über die Hülse verbundenen Bolzenpaares bilden daher die Anbringungspunkte für eine Pedikelschraube. Durch Veränderung des axialen Abstands der Bolzen zueinander, z.B. mit Hilfe einer Gewindehülse, lassen sich daher die Anbringungspunkte verstellen. Gleichzeitig ermöglichen die Bolzen eine Rotation der Pedikelschrauben um die Achse der Bolzen um einen gewünschten Winkel. Die Pedikelschrauben können darüber hinaus um eine Achse senkrecht zu ihrer Achse gegenüber den Verbindungspunkten verschwenkt und in der eingenommenen Position festgelegt werden. Die Pedikelschraube kann daher im Raum jede gewünschte Lage einnehmen und in dieser Lage festgesetzt werden.

Mit der erfindungsgemäßen Stützvorrichtung lassen sich daher die Pedikelschrauben in jeden gewünschten Wirbel einschrauben und gewünschte Wirbel eines Wirbelsäulenabschnitts gegeneinander festlegen. Die erfindungsgemäße Stützvorrichtung ist daher für eine primäre Stabilisierung der Wirbelsäule, etwa im Fall von Frakturen eines oder mehrerer Wirbel geeignet. Die miteinander verbundenen, eine Haltevorrichtung bildenden Bolzen können ausreichend stabil ausgeführt werden, um die gewünschte Kraftaufnahme zu gewährleisten und etwa frakturierte Wirbel zu entlasten. Die erfindungsgemäße Stützvorrichtung ist jedoch auch für die Korrektur mehr oder weniger langer Wirbelsäulenabschnitte geeignet.

Um eine möglichst wirksame Festlegung der Bolzenpaare gegeneinander und der Pedikelschrauben gegenüber den Bolzenköpfen zu erzielen, weisen die Klemmflächen eine möglichst hohe Rauhigkeit auf, damit ein hoher Kraftschluß erzielt wird. Alternativ können nach einer bevorzugten Ausgestaltung der Erfindung die Klemmflächen eine Zahnung aufweisen. Die Zahnung ermöglicht eine stufenweise Verstellung der Klemmflächen gegeneinander im Mindestabstand eines Zahnabstands, der jedoch für die meisten vorkommenden Fälle völlig ausreichen dürfte. Vor allen Dingen wird jedoch ein Formschluß erzielt, durch den eine hohe Verdrehsicherung gewährleistet ist.

Die Klemmflächen werden, wie bereits erwähnt, mit Hilfe einer Schraubverbindung gegeneinandergepreßt. Vorzugsweise wird ein Schraubenbolzen vorgesehen, der sich durch Bohrungen der Bolzenköpfe und der Pedikelschrauben hindurcherstreckt. Die Schraubenbolzen wirken vorzugsweise mit einer Gewindebohrung in einem Bolzenkopf zusammen, wobei ein zweiter Bolzenkopf bzw. die Bohrung im Kopf der Pedikelschraube einen Innenkonus aufweisen, der mit dem Konus des Schraubenbolzenkopfes zusammenwirkt, um eine wirksame Kraftübertragung zu gewährleisten. Außerdem hat eine derartige Ausbildung den Vorteil, daß der Schraubenbolzen völlig versenkt anbringbar ist und mithin kein störend hervortretendes Teil bildet.

Wie bereits erwähnt, kann der Abstand der Bolzenköpfe über die Gewindehülse eingstellt werden, wenn die Bolzen ein Gewinde haben. Diese weist nach einer weiteren Ausgestaltung der Erfindung Schlüsselflächen auf. Auf diese Weise kann eine erhebliche axiale Kraft aufgebracht werden, um zum Beispiel zwei Wirbel relativ zueinander zu verstellen. In erster Linie dient die Hülse jedoch dazu, die Bolzenköpfe an die jeweiligen Abmessungen der Wirbel anzupassen.

Um eine eingenommene Lage der Bolzenköpfe zueinander zu fixieren und auch deren axialen Abstand, sind geeignete Feststellmittel vorgesehen. Diese bestehen nach einer Ausgestaltung der Erfindung darin, daß eine Kontermutter auf ein Gewinde eines Bolzens aufgeschraubt ist. Die Kontermutter sichert die Drehlage des Bolzens relativ zur Gewindehülse und damit auch deren axiale Lage. Alternativ oder zusätzlich kann die Gewindehülse mit zwei Feststellschrauben versehen werden, um diese drehfest gegenüber dem Gewindeschaft der Gewindebolzen zu fixieren.

Eine andere Möglichkeit, die Hülse gegenüber den Bolzen festzusetzen, besteht nach einer Ausgestaltung der Erfindung darin, daß eine durch Quetschung verformbare Hülse vorgesehen ist. Ist der Abstand der Bolzenköpfe richtig eingestellt, kann die Hülse auf die Bolzen aufgequetscht werden, die nach einer anderen Ausgestaltung der Erfindung mit Abflachungen an den Bolzenenden zusammenwirkt, um eine wirksame Drehsicherung zu erzielen. Mit Hilfe von Bunden oder Nuten läßt sich auch eine axiale Sicherung durch Quetschen erreichen. Eine aufquetschbare Hülse kann sowohl als Gewinde- als auch als glatte Hülse ausgebildet sein, um eine Verbindung zwischen den Bolzen und ein anschließendes Festsetzen zu bewerkstelligen. Falls die Hülse nicht mit Gewinde versehen ist, kann sie von vornherein fest mit einem Bolzen verbunden werden, so daß lediglich eine Relativbewegung zwischen dem anderen Bolzen und der Hülse möglich ist, so daß eine Quetschverbindung nur mit dem beweglichen Bolzen hergestellt werden muß.

Bei einer alternativen Ausgestaltung der Erfindung ist vorgesehen, daß die Hülse zumindest teilweise geteilt ist und der Hülse eine Klemmvorrichtung zugeordnet ist zum Festklemmen der Hülse auf mindestens einem Bolzen. In diesem Fall ist die Hülse als Klemmring oder eine Art Schelle ausgebildet, die mit Hilfe einer Klemmvorrichtung, beispielsweise einer Schraubverbindung, auf einem oder beiden Bolzen klemmend befestigbar ist.

Eine besonders wirksame Drehsicherung wird dadurch erhalten, daß der Gewindebolzen mindestens eine Abflachung aufweist. Auf dem Gewindebolzen sitzt eine Scheibe, die aufgrund der Abflachung drehfest, jedoch axial verschiebbar ist. Die Scheibe weist eine stirnseitige Verzahnung auf, vergleichbar der Verzahnung für die bereits beschriebene Pedikelschraube. Eine entsprechende Zahnung ist auch am Stirnende der Hülse vorgesehen. Mit Hilfe der Kontermutter wird die Scheibe gegen die Hülse angepreßt. Auf diese Weise wird eine doppelte Drehsicherung erhalten. Die eine findet zwischen der Scheibe und der Gewindehülse statt und die zweite zwischen der Kontermutter und der Scheibe.

Als Stützvorrichtung für die Wirbelsäule ist zum Beispiel ein sogenannter Harrington-Stab bekanntgeworden, der an einem Ende an einem Wirbel der Wirbelsäule angesetzt werden kann und der im anderen Endbereich eine stufenweise Verstellung eines Halteglieds erlaubt, das mit einem anderen Wirbel verbindbar ist. Die erfindungsgemäße Stützvorrichtung kann auch mit einem derartigen Distraktionsstab kombiniert werden. In diesem Zusammenhang sieht eine Ausgestaltung der Erfindung vor, daß mindestens ein separater länglicher Bolzen vorgesehen ist mit einem eine Klemmfläche aufweisenden Kopf sowie mit Haltemitteln am anderen Ende für ein an einem Wirbel anzusetzendes Anschlußglied. Bei dieser Ausführungsform hat zum Beispiel der Harrington-Stab an dem den Haltemitteln entgegengesetzten Ende ein Auge mit der erfindungsgemäßen Klemmfläche. Bei einer anderen Ausgestaltung der Erfindung weist der Bolzenschaft eine Sackbohrung auf, in die der zylindrische Zapfen eines länglichen Distraktionsstabes, beispielsweise ebenfalls eines Harrington-Stabes, einsteckbar ist. Eine Auszugssicherung muß nicht vorgesehen werden, da eine derartige Stützvorrichtung normalerweise auf Druck belastet ist.

Eine dritte Alternative sieht vor, daß die bereits mehrfach erwähnte Hülse mit einer Einsteckbohrung versehen ist zur Aufnahme eines vorzugsweise zylindrischen Zapfens eines länglichen Stabes, beispielsweise eines Harrington-Stabes.

Mit einem Harrington-Stab oder dergleichen können auch Haken kombiniert werden, die in einem Halteabschnitt eine Durchbohrung aufweisen. Mit Hilfe einer Feststellschraube kann ein Haken in einer beliebigen axialen Lage auf dem Stab fixiert werden. Der Haken kann beispielsweise ein sogenannter Laminahaken sein, der unter die Lamina eines Wirbels greift. Er kann jedoch jedoch auch ein Pedikelhaken sein, der vorzugsweise am freien Ende gabelförmig ausgeführt ist. Ein unterer Laminahaken kann ebenfalls mit einer Klemmfläche versehen werden, die mit der Klemmfläche des länglichen Stabes zusammenwirkt. Der Laminahaken kann zusätzlich mit einer Lasche ausgestattet werden, um ihn an einem Wirbelkörper mit Hilfe von Knochenschrauben zu fixieren. Zwei nebeneinander, jedoch im Abstand verlaufende Stäbe können mit Hilfe von Doppelhaken zusammengespannt werden, wobei die Doppelhaken mit Hilfe geeigneter Mittel in einem gewünschten Abstand voneinander feststellbar sind.

Wie bereits erwähnt, ist bekannt, Pedikelschrauben mit Hilfe von Muttern auf einer Gewindestange festzulegen. Die erfindungsgemäße Stützvorrichtung kann auch mit der bekannten Stützvorrichtung kombiniert werden. Hierzu sieht eine Ausgestaltung der Erfindung einen Adapter vor, der an einem Ende eine Klemmfläche aufweist zur Verbindung mit einem Bolzen oder einer Pedikelschraube mittels der Schraubverbindung. Der Adapter weist ferner einen Gabelabschnitt im Abstand zur Klemmfläche auf zur Aufnahme eines länglichen, vorzugsweise ein Gewinde aufweisenden Stabes in einer Ebene annähernd parallel zu der durch die benachbarte Pedikelschraube aufgespannten Ebene, wobei der Stab vorzugsweise mittels Muttern am Adapter festlegbar ist. Bei dieser Ausführungsform ist die Achse des Stabes gegenüber der Bolzenachse seitlich versetzt. Dieser seitliche Versatz ist jedoch unkritisch.

Die Erfindung wird nachfolgend anhand eines in Zeichnungen darstellten Ausführungsbeispiels näher erläutert.
- Fig. 1: zeigt die Draufsicht auf einen schematisch dargestellten Lendenwirbel und einen Brustwirbel.
- Fig. 2: zeigt eine Draufsicht, teilweise im Schnitt auf die erfindungsgemäße Stützvorrichtung.
- Fig. 3: zeigt eine Ansicht der Stützvorrichtung nach Fig. 2 in Richtung Pfeil 3, jedoch mit einer besonderen Drehsicherung.
- Fig. 4: zeigt einen Schnitt durch die Darstellung nach Fig. 3 entlang der Linie 4-4.
- Fig. 5: zeigt eine Seitenansicht eines steckbaren Bolzens.
- Fig. 6: zeigt eine um 90° verdrehte Seitenansicht des Bolzens nach Fig. 5.
- Fig. 7: zeigt einen längeren stabförmigen Bolzen, der als Harrington-Stab geformt ist.
- Fig. 8: zeigt die Draufsicht auf eine Verbindung eines Bolzens mit einem herkömmlichen Gewindestab.
- Fig. 9: zeigt einen Schnitt durch die Darstellung nach Fig. 8 entlang der Linie 9-9 um 90° verdreht.
- Fig. 10: zeigt einen Schnitt durch den in den Figuren 8 und 9 gezeigten Adapter.
- Fig. 11: zeigt eine abgewandelte Form eines Harrington-Stabes mit einem oberen Laminahaken.
- Fig. 12: zeigt den Haken nach Fig. 11 in umgekehrter Anordnung auf dem Stab.
- Fig. 13: zeigt eine Draufsicht auf einen Teil des oberen Pedikelhakens nach Fig. 12.
- Fig. 14: zeigt einen unteren Laminahaken zur Anbringung an der Klemmfläche des Stabes nach Fig. 11.
- Fig. 15: zeigt einen oberen Pedikelhaken mit zusätzlicher Lasche.
- Fig. 16: zeigt einen Querverbindungshaken.

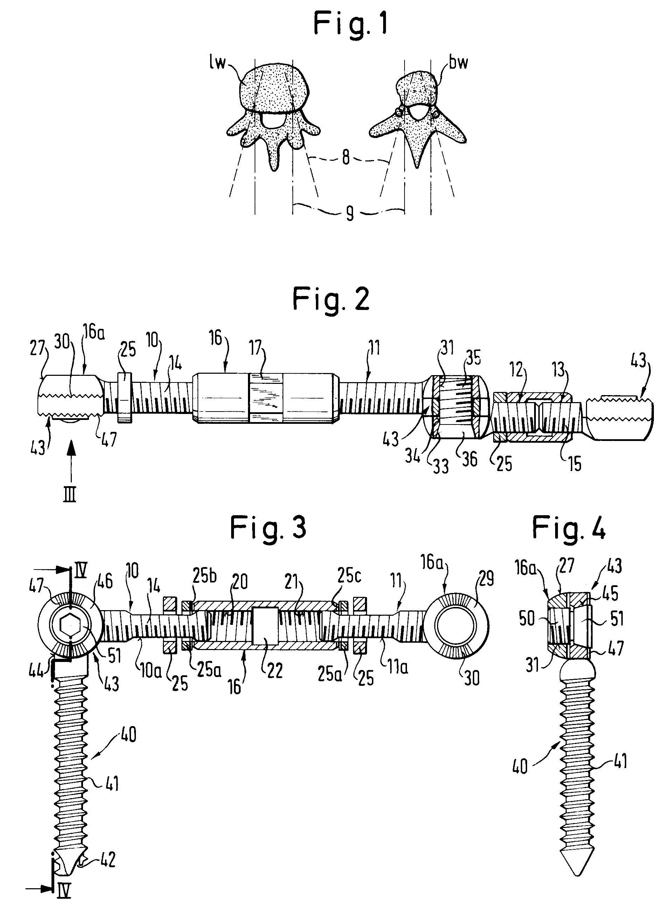

Fig. 1 veranschaulicht einen Lendenwirbel lw bzw. einen Brustwirbel bw in schematischer Draufsicht. Die gestrichelten Linien 10 deuten Achsen von Bohrungen an, die zur Aufnahme von Pedikelschrauben vorgenommen werden. Die Bohrungen erstrecken sich zwischen Dornfortsatz und Querfortsatz bzw. Rippenfortsatz durch den Wirbelbogenfuß (Pedikel) in den Wirbelkörper hinein. Strichpunktiert sind Achsen dargestellt ebenfalls für Bohrungen durch die Pedikel in den Wirbelkörper hinein wie sie zur Aufnahme von Pedikelschrauben vorgenommen werden können.

Die in den Figuren 2 bis 4 dargestellte Stützvorrichtung weist Gewindebolzen 10, 11 bzw. 12, 13 auf. Sie sind paarweise angeordnet und weisen einen Gewindeschaft 14 bzw. 15 und einen Kopf 16a auf. Wie erkennbar, ist der Gewindeschaft 14 der Gewindebolzen 10, 11 länger als der Gewindeschaft 15 der Gewindebolzen 12, 13. Der Kopf 16a ist bei allen Gewindebolzen außen von gleichen Abmessungen. Die Gewindeschäfte 14 der Gewindebolzen 10, 11 sind über eine Gewindehülse 16 miteinander verbindbar. Die Gewindehülse 16 ist in der Mitte mit einem Sechskant 17 versehen, der Schlüsselflächen bildet. Die Gewindehülse 16 weist innen zwei Gewindebohrungsabschnitte 20, 21 auf, die gegenläufiges Gewinde aufweisen. Dazwischen befindet sich ein im Durchmesser erweiterter Entlastungsbohrungsabschnitt 22. Durch Drehung der Gewindehülse 16 können die Gewindebolzen 10, 11 in ihrem axialen Abstand voneinander verändert werden.

Auf dem Gewindeschaft 14 des Gewindebolzens 10 in Fig. 2 sitzt eine Kontermutter 25, die gegen das zugeordnete Stirnende der Gewindehülse anlegbar ist. Es versteht sich, daß auf dem Gewindeschaft des Gewindebolzens 11 ebenfalls eine Kontermutter angeordnet sein kann.

Bei der abgewandelten Ausführungsform nach Fig. 3 sind die Schäfte 14 der Gewindebolzen 10, 11 mit diametral gegenüberliegenden Abflachungen 10a, 11a versehen. Auf den Gewindebolzen 10, 11 sitzen Scheiben 25a, deren Öffnung dem Querschnitt der Gewindebolzen 10, 11 angepaßt ist. Sie können daher axial verschoben, jedoch nicht relativ zum Gewindebolzen verdreht werden. Die Scheiben 25a haben auf einer Stirnseite eine Verzahnung 25b entsprechend den Köpfen 16a der Gewindebolzen (auf die Verzahnung wird weiter unten noch näher eingegangen). Die Enden der Gewindehülse 16 sind mit einer entsprechenden Verzahnung 25c versehen, die mit der Verzahnung 25b zusammwirkt, um die Gewindehülse an einer Drehung gegenüber der Scheibe 25b zu hindern.

Die Gestalt des Kopfes 16a der Gewindebolzen ist ringförmig und außen leicht ballig, wie bei 27 gezeigt. Auf der im Durchmesser größeren Seite des ringförmigen Kopfes 16 ist eine ringförmige Klemmfläche 29 vorgesehen, die im wesentlichen durch eine Zahnung 30 gebildet ist. Wie aus Fig. 2 erkennbar, weist der Kopf des Gewindebolzens 11 eine Gewindebohrung 31 auf. Der Kopf des Gewindebolzens 12 weist hingegen einen konischen Bohrungsabschnitt 33 auf, an den sich ein zylindrischer Bohrungsabschnitt 34 anschließt. Die beschriebenen Abschnitte nehmen einen Schraubenbolzen 35 auf, der einen konischen Kopf 36 aufweist, der mit dem konischen Bohrungsabschnitt 33 des Gewindebolzens 12 zusammenwirkt.

Die gezeigte Stützvorrichtung weist auch Pedikelschrauben auf, von denen eine bei 40 in Figuren 3 und 4 dargestellt ist. Die Pedikelschraube 40 weist einen Gewindeschaft 41 auf mit einem selbstschneidenden Gewinde, das an der Spitze eine Schneidnut 42 aufweist. Das Gewinde läßt nahe dem ringförmigen Kopf 43 einen glattzylindrischen Abschnitt 44 frei. Wie in den Figuren 2 und 4 zu erkennen, weist der Kopf 43 der Pedikelschraube 40 eine doppelkonische Bohrung 45 auf. Auf beiden Seiten sind ringförmige Klemmflächen 46 vorgesehen, die von einer Zahnung 47 gebildet wird. Die Zahnung 47 wirkt mit der Zahnung 30 der Klemmflächen 29 der Köpfe 16a der Gewindebolzen 10 bis 13 zusammen. In Fig.2 ist dargestellt, wie der Kopf 43 einer Pedikelschraube 40 zwischen zwei Klemmflächen 29 der Kopfe 16a zweier benachbarter Gewindebolzen 12 und 11 eingespannt ist, und zwar mit Hilfe des Schraubenbolzens 35. In Figuren 3 und 4 ist dargestellt, wie eine Pedikelschraube 40 mit Hilfe eines Schraubenbolzens 50, der einen konischen Kopf 51 aufweist, gegen einen Kopf 16a des Gewindebolzens 11 festgespannt wird. Der Gewindebolzen 50, der ebenfalls völlig versenkt anbringbar ist, gleicht im Aussehen dem Gewindebolzen 35, ist jedoch kürzer als dieser. Der Kopf der Gewindebolzen weist innere versenkte Schlüsselflächen auf, beispielsweise einen Innensechskant, um die gewünschte Verschraubung zu bewerkstelligen.

Die Pedikelschrauben 40 werden in der oben beschriebenen Weise in die gewünschten Wirbel eingesetzt. Mit Hilfe der längeren Gewindebolzen 10, 11 ist es möglich, zwei um einen Wirbel beabstandete Wirbel miteinander zu verbinden bzw. gegeneinander abzustützten, wobei der genaue Abstand mit Hilfe der Gewindehülse 16 eingestellt werden kann. Die Gewindebolzen 12, 13 ermöglichen hingegen eine Verbindung von zwei benachbarten Wirbeln mit Hilfe eingeschraubter Pedikelschrauben 40. Wie erkennbar, kann die Drehlage um die Bohrungsachse des Kopfes 43 der Pedikelschrauben 40 im Abstand der Zähne der Zahnung 47 variiert werden. Die Änderung der Drehlage der Pedikelschrauben 40 senkrecht dazu kann durch Verdrehung der zugeordneten Gewindebolzen 10, 11, 12, 13 erfolgen. Der Abstand benachbarter Pedikelschrauben läßt sich über die Gewindehülse 16 verstellen. Durch das Zusammenpressen der Klemmflächen und das Festlegen der Gewindehülse 16 mit Hilfe der Kontermuttern 25 bzw. der Scheiben 25a läßt sich daher eine beliebig im Raum eingestellte Position der Pedikelschrauben 40 festlegen. Dies gilt für jede Pedikelschraube, die in der Kette aneinandergereihter Gewindebolzenpaare angebracht ist. Es versteht sich, daß jeweils zwei derartige Ketten entlang der Wirbelsäule bzw. eines Wirbelabschnitts vorgesehen werden müssen.

Die beschriebene Stützvorrichtung ist modulartig aufgebaut, wobei jedoch miteinander verbundene Köpfe von Gewindebolzen unterschiedliche Bohrungen aufweisen, von denen eine eine Gewindebohrung und die andere einen konischen Bohrungsabschnitt aufweist. Die Pedikelschrauben hingegen können für alle Gewindebolzen gleich ausgeführt werden.

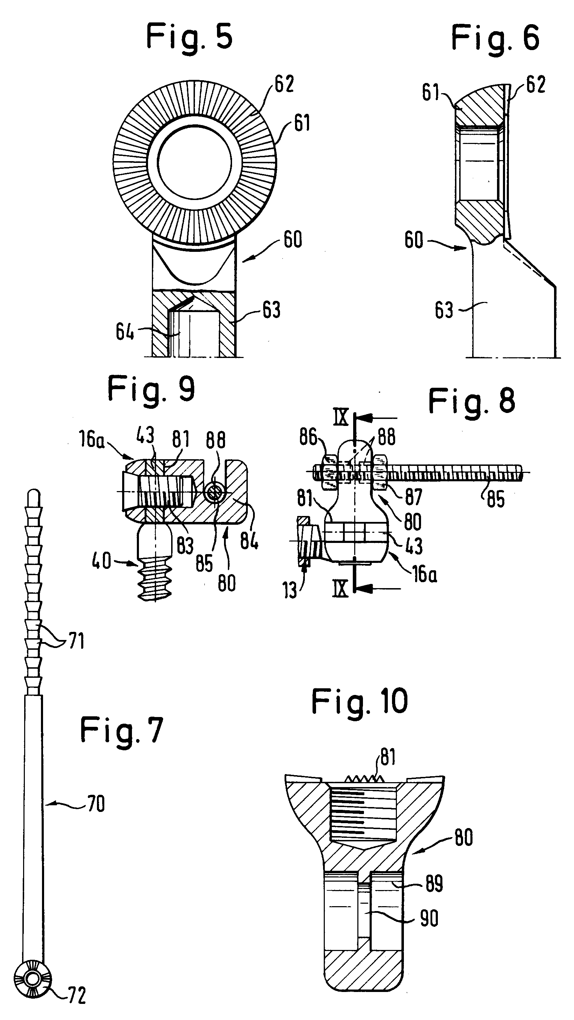

In den Figuren 5 und 6 ist ein Bolzen 60 dargestellt, der einen ringförmigen Kopf 61 aufweist. Der ringförmige Kopf 61 entspricht dem Kopf 16a nach den Figuren 2 bis 4 mit folgenden Ausnahmen. Seine gezahnte Klemmfläche 62 ist leicht kegelscheibenförmig, wie aus Fig. 6 hervorgeht und liegt annähernd auf der Achse des Bolzenschaftes 63. Der Bolzenschaft 63 weist eine Sackbohrung 64 auf. Sie dient zum Beispiel zur Aufnahme eines zylindrischen Zapfens eines nicht gezeigten länglichen Stabes, zum Beispiel eines Harrington-Stabes.

Fig. 7 zeigt einen modifizierten Harrington-Stab 70, der in einem Endbereich mit einer Reihe von konischen Abschnitten 71 versehen ist, die mit einem ringförmigen Anschlußstück zusammenwirken, das an einem Wirbel ansetzbar ist. Bei der Anbringung rutscht das Anschlußstück die konischen Abschnitten 21 entlang, wenn die gekrümmte Wirbelsäule geradegebogen wird, bis es hinter dem letzten konischen Abschnitt 71 verrastet. Am anderen Ende weist der Harrington-Stab 70 einen ringförmigen Kopf 72 auf, der dem Kopf 61 nach den Figuren 5 und 6 oder dem Kopf 16a nach den Figuren 2 bis 4 gleicht. Einzelheiten müssen daher nicht mehr beschrieben werden. Mittels des Kopfes 72 kann daher ein Anschluß zum Beispiel an einen Bolzen 10 oder 11 nach den Figuren 2 bis 4 erfolgen mit oder ohne eine der in den Figuren 2 bis 4 dargestellten Pedikelschrauben.

Bei der Ausführungsform nach den Figuren 8 und 9 ist das Ende eines Bolzens 13 gezeigt mit dem Kopf 16a entsprechend der Darstellung nach Fig. 2 in Verbindung mit einer Pedikelschraube 40. Auf der gegenüberliegenden Seite des Kopfes 43 der Pedikelschraube 40 ist ein Adapter 80 angeordnet, der eine Klemmfläche 81 aufweist, die den Klemmflächen 46 bzw. 29 der Pedikelschraube 40 bzw. des Bolzens 11 entspricht. Der Adapter 80 weist auch eine Sackbohrung auf zur Aufnahme eines Schraubenbolzens 83, der im Prinzip dem Schraubenbolzen 35 nach Fig. 2 zumindest im Hinblick auf die Wirkungsweise gleicht. Der Adapter 80 weist einen gabelförmigen Abschnitt 84 auf, der einen Gewindestab 85 aufnimmt. Der Gewindestab 85 ist in seinem Durchmesser etwas kleiner als die Breite des Gabelspaltes. Auf der Gewindestange 85 sitzen zwei Muttern 86, 87, die einen zylindrischen Abschnitt 88 aufweisen, deren Außendurchmesser der Breite des Spaltes des Gabelabschnitts 84 ist. Mit Hilfe der Muttern 86, 87 wird die Gewindestange 85 im Gabelabschnitt 84 festgelegt. Aus Fig. 10 ist zu erkennen, daß der Gabelspalt 89 eine Verengung 90 aufweisen kann, wobei die Verengung dem Durchmesser der Gewindestange 85 entspricht, während die übrige Breite, wie bereits erwähnt, dem Durchmesser der zylindrischen Abschnitte 88 der Muttern 86, 87 entspricht.

In den Figuren 8 und 9 ist zu erkennen, daß die Achse der Gewindestange 85 gegenüber der Achse des Bolzens 13 etwas versetzt liegt. Dieser Versatz ist jedoch unkritisch.

Die Pedikelschraube 40 kann einen konischen Kern aufweisen, der sich zum freien Ende hin verjüngt.

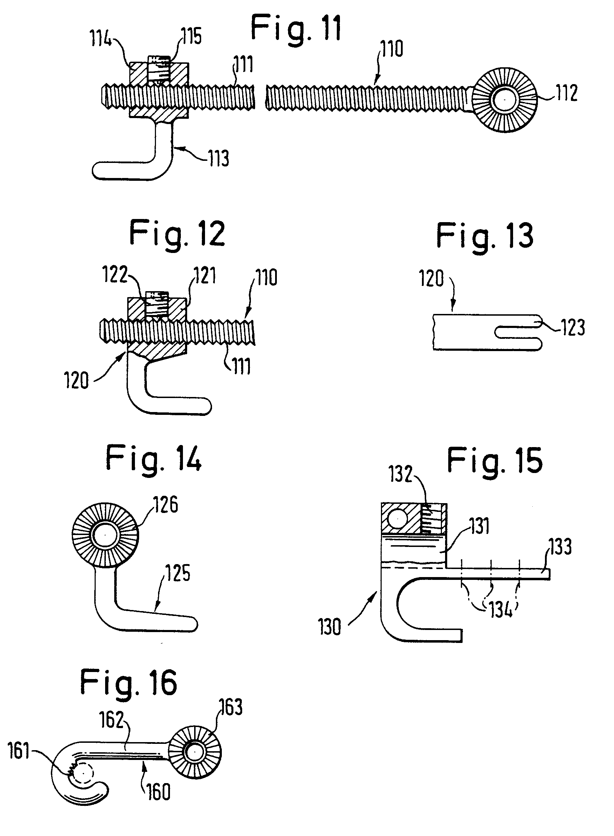

In Fig. 11 ist ein Distraktionsstab nach Fig. 7 dargestellt. Sein länglicher Schaft 110 weist eine schraubenlinienförmig genutete Oberfläche 111 auf. An einem Ende ist ein ringförmiger Klemmkopf 112 angeformt mit einer radialen Verzahnung, die in Verbindung mit den anderen Figuren bereits beschrieben wurde. Ein Laminahaken 113, der unter die Lamina eines Wirbels faßt, weist einen Halteabschnitt 114 auf mit einer Durchbohrung, durch die der Schaft 110 hindurchführbar ist. Mit Hilfe einer Feststellschraube 115, die in einer Gewindebohrung quer zur Durchbohrung des Halteabschnitts 114 verläuft, kann der Haken 113 an einer gewünschten axialen Position am Stab 110 fixiert werden. Die Schraube 115, die als Madenschraube mit zum Beispiel Innensechskant ausgeführt ist, kann am unteren Ende spitz auslaufen, um mit der gewindeartigen Oberfläche 111 zusammenzuwirken. Diese verhindert, daß der Haken 113 auf der Stange 110 rutscht. Der Klemmkopf 112 kann zum Beispiel mit Hilfe einer Pedikelschraube in gewünschter Orientierung an einem unteren Wirbel befestigt werden.

In Fig. 12 ist das Ende des Stabs 110 nach Fig. 11 dargestellt. In diesem Fall dient er zur Aufnahme eines oberen Pedikelhakens 120, der einen Halteabschnitt 121 aufweist mit einer Gewindebohrung zur Aufnahme einer Feststellschraube 122. Während die Hakenöffnung des Laminahakens 113 weist von dem Klemmkopf 112 fort; die Hakenöffnung des Pedikelhakens 120 weist in die gleiche Richtung. Der Haken 120 umgreift den Pedikel eines Wirbels. Er kann, wie in Fig. 13 dargestellt, zum freien Ende hin gabelförmig ausgeführt sein, wie bei 123 gezeigt.

Fig. 14 zeigt einen unteren Laminahaken 125, der am der Hakenspitze abgewandten Ende einen Klemmkopf 126 aufweist. Der Haken kann zum Beispiel mit dem Klemmkopf 112 der Stange 110 in gewünschter Winkelbeziehung angebracht werden. Die unterschiedliche Winkelanordnung ermöglicht eine günstige anatomische Anpassung und verhindert, daß der Haken von der Lamina eines unteren Wirbels abrutscht, was eine Reoperation erforderlich machen würde.

Häufig ist erforderlich, zwei stabartige Fixationselemente, die mehr oder weniger parallel zur Wirbelsäule geführt sind, gegeneinander zu verspannen. Es besteht die Gefahr, daß konventionelle mit dem Stab verbundene Haken herausreißen. In Fig. 15 ist ein Pedikelhaken gezeigt, der einer derartigen Belastung standhält. Der Pedikelhaken 130 entspricht in seinem Aufbau etwas dem nach den Figuren 12 und 13. Der Stab 110 nach Fig. 11 wird durch eine Durchbohrung 131 hindurchgesteckt, und eine quer dazu verlaufende Gewindebohrung 132 nimmt eine Feststellschraube auf, um den Haken 130 in einer gewünschten axialen Position am Stab 110 zu fixieren. Zusätzlich weist der Haken 130 parallel zur Achse der Durchbohrung 131 eine flache Lasche 133 auf mit drei Durchbohrungen, die lediglich durch strichpunktierte Linien 134 angedeutet sind. Sie dienen zur Aufnahme von Knochenschrauben, um den Pedikelhaken 130 zusätzlich am Wirbelkörper festzulegen.

Mit dem beschriebenen Haken und den Stangen läßt sich in gewünschter Weise eine Distraktion oder Kompression vornehmen je nach den vorliegenden Gegebenheiten.

Bei der Ausführungsform nach Fig. 16 weist ein Haken 160 zum Umfassen eines zum Beispiel Distraktionsstabes (gestrichelt angedeutet) eine Zahnung 161 auf - um auch eine axiale Lage des Stabes zu fixieren. Auf der dem Haken gegenüberliegenden Seite ist am Halteabschnitt 162 ein Klemmkopf 163 angeformt,vergleichbar dem Klemmkopf 112 für den Stab 110 nach Fig. 11. Der Klemmkopf 163 dient zum Beispiel dazu, mit einer Pedikelschraube gemäß Fig. 4 verspannt zu werden.

Der oben gezeigte, modifizierte Distraktionsstab kann nicht nur durch die Lamina- oder Pedikelschrauben geschoben werden, sondern der Stab kann auch in die Haken eingelegt werden und mit einer entsprechend angebrachten Klemmvorrichtung fixiert werden. Dies ist notwendig, wenn ein am unteren oder oberen Ende fixierter Stab noch in seinem Mittelteil über zusätzliche Haken fixiert werden soll. Aus implantationstechnischen Gründen ist das Einbringen von zusätzlich am Stab angebrachten Haken vorher nicht möglich.

## Patentansprüche

1. Stützvorrichtung für die menschliche Wirbelsäule, mit einer langgestreckten, dorsal implantierbaren Haltevorrichtung, mindestens zwei einen Kopf (43) und einen Gewindeschaft (41) aufweisenden Pedikelschrauben (40), deren Kopf (43) schwenkbar um eine Achse an der Haltevorrichtung gelagert ist, die annähernd senkrecht zur Gewindeschaftachse verläuft, während der Gewindeschaft (41) von der Haltevorrichtung absteht, und einer Befestigungsvorrichtung (25, 25a) für die Fixierung der Pedikelschrauben (40) in der eingestellten Winkellage und in gewünschten axialen Abständen voneinander, dadurch gekennzeichnet, daß die Haltevorrichtung mindestens zwei getrennte, vorzugsweise ein Gewinde aufweisende Bolzen (10, 11; 12, 13) aufweist, die mit einer ein Gewinde aufweisenden Hülse (16) zusammenwirken und die an den Kopfenden (16a) eine auf der Achse oder achsparallel liegende Klemmfläche (29) aufweisen, der Kopf (43) der Pedikelschrauben (40) zwei annähernd parallele, sich annähernd parallel zur Gewindeschaftachse erstreckenden Klemmflächen (46) aufweist und je Bolzen (10, 11; 12, 13) eine Schraubverbindung (35, 50) vorgesehen ist, die die Klemmflächen von Bolzen und Pedikelschrauben oder von zwei Bolzen in ihrer Lage klemmend gegeneinander festlegt.

2. Stützvorrichtung nach Anspruch 1, dadurch gekennzeichnet, daß die Klemmflächen (29; 46) eine große Rauhigkeit aufweisen.

3. Stützvorrichtung nach Anspruch 1, dadurch gekennzeichnet, daß die Klemmflächen (29; 46) eine Zahnung (30; 47) aufweisen.

4. Stützvorrichtung nach einem der Ansprüche 1 bis 3, dadurch gekennzeichnet, daß die Klemmflächen (29) der Bolzen (10, 11; 12, 13) von Bohrungen (31, 33, 34) durchsetzt und von den Schraubenbolzen (35, 50) aufgenommen sind.

5. Stützvorrichtung nach Anspruch 1, dadurch gekennzeichnet, daß die Gewindehülse (16) Schlüsselflächen (17) aufweist.

6. Stützvorrichtung nach einem der Ansprüche 1 bis 5, dadurch gekennzeichnet, daß die Befestigungsvorrichtung mehrere mit einem Gewinde der Bolzen (10, 11, 12, 13) und der Gewindehülse (16) zusammenwirkende Feststellmittel (25, 25a, 25b, 25c) zur Festlegung der axialen und Drehlage dieser Teile aufweist.

7. Stützvorrichtung nach Anspruch 6, dadurch gekennzeichnet, daß eine Kontermutter (25) auf dem Gewinde der Bolzen (10, 11; 12, 13) sitzt.

8. Stützvorrichtung nach Anspruch 6, gekennzeichnet durch eine durch Quetschung verformbare Gewindehülse (16) und mindestens eine Abflachung an dem in der Gewindehülse einsitzenden Abschnitt des Bolzens.

9. Stützvorrichtung nach Anspruch 6,dadurch gekennzeichnet, daß die Gewindehülse zumindest teilweise geschlitzt ist und der Gewindehülse eine Klemmvorrichtung zugeordnet ist zum Festklemmen der Gewindehülse auf mindestens einem Bolzen.

10. Stützvorrichtung nach Anspruch 6 und 7, dadurch gekennzeichnet, daß der Bolzen (10, 11) mindestens eine Abflachung (11a) aufweist, zwischen Kontermutter (25) und Gewindehülse (16) eine Scheibe (25a) unverdrehbar auf dem Bolzen (10, 11) sitzt, die eine stirnseitige Zahnung (25b) aufweist, die mit einer stirnseitigen Zahnung (25c) der Gewindehülse (16) zusammenwirkt.

11. Stützvorrichtung nach einem der Ansprüche 1 bis 10,dadurch gekennzeichnet, daß die Klemmflächen (29; 46) kreisförmig sind.

12. Stützvorrichtung nach einem der Ansprüche 1 bis 11,dadurch gekennzeichnet, daß zwischen dem Kopf (43) und dem Gewindeschaft (41) der Pedikelschrauben (40) ein glattzylindrischer Abschnitt (44) vorgesehen ist.

13. Stützvorrichtung nach einem der Ansprüche 1 bis 12,dadurch gekennzeichnet, daß der Gewindeschaft (41) der Pedikelschrauben (40) einen konischen sich zum freien Ende hin verjüngenden Kern aufweist.

14. Stützvorrichtung nach einem der Ansprüche 4 bis 12,dadurch gekennzeichnet, daß der Kopf (36, 51) des Schraubenbolzens (35, 50) konisch ist und mit einem konischen Bohrungsabschnitt (34, 45) der Pedikelschrauben (40) bzw. der Bolzen (10, 11) zusammenwirkt.

15. Stützvorrichtung nach Anspruch 14, dadurch gekennzeichnet, daß der Bohrungsabschnitt (45) der Pedikelschrauben (40) doppelkonisch ist.

16. Stützvorrichtung nach Anspruch 14 oder 15, dadurch gekennzeichnet, daß der Schraubenbolzenkopf (36, 51) versenkte Schlüsselflächen aufweist.

17. Stützvorrichtung nach einem der Ansprüche 1 bis 16,dadurch gekennzeichnet, daß mindestens ein separater, längerer Distraktionsstab (70; 110) vorgesehen ist mit einem eine Klemmfläche aufweisenden Kopf (72; 112), die mit der Klemmfläche (30) des Bolzens (10a) zusammenwirkt sowie mit Haltemitteln (71; 115) am anderen Ende für ein an einem Wirbel anzusetzendes Anschlußglied.

18. Stützvorrichtung nach einem der Ansprüche 1 bis 16,dadurch gekennzeichnet, daß der Schaft (63) eines Bolzens (60) mit einer axialen Sackbohrung (64) versehen ist zur Aufnahme eines vorzugsweise zylindrischen Zapfens eines längeren Stabes, der am anderen Ende mit Haltemitteln versehen ist für ein an einem Wirbel anzusetzendes Anschlußglied, wobei der Kopf (62) des Bolzens (60) mit Sackbohrung (64) dem Kopf (16a) des Bolzens (10) gleicht.

19. Stützvorrichtung nach einem der Ansprüche 1 bis 18,dadurch gekennzeichnet, daß der Kopf des Bolzens eine Einsteckbohrung aufweist zur Aufnahme eines vorzugsweise zylindrischen Zapfens eines längeren Distraktionsstabes, der an einem Ende mit Haltemitteln versehen ist für ein an einem Wirbel anzusetzendes Anschlußglied.

20. Stützvorrichtung nach einem der Ansprüche 1 bis 19,gekennzeichnet durch einen Adapter (80), der an einem Ende eine Klemmfläche (81) aufweist zur Verbindung mit einem Bolzen und/oder einer Pedikelschraube (40) mittels der Schraubverbindung (83) sowie einen Gabelabschnitt (84) im Abstand zur Klemmfläche (81) zur Aufnahme eines länglichen vorzugsweise ein Gewinde aufweisenden Stabes (85), wobei die Achse des Stabes in einer gegenüber der Achse des Bolzens hindurchgehenden Ebene versetzten Ebene liegt und wobei der Stab (85) mittels Muttern (86, 87) am Adapter (80) festlegbar ist.

21. Stützvorrichtung nach Anspruch 17, dadurch gekennzeichnet, daß das Anschlußglied ein Haken (120) ist, der einen Halteabschnitt (121) aufweist mit einer Durchbohrung, durch die ein Distraktionsstab (110) hindurchgeführt ist und der Halteabschnitt (121) mittels einer Feststellschraube (122) am Stab (110) befestigbar ist.

22. Stützvorrichtung nach Anspruch 21, dadurch gekennzeichnet, daß das Ende des Hakens (120) gabelförmig (123) ausgebildet ist.

23. Stützvorrichtung nach Anspruch 21 oder 22, dadurch gekennzeichnet, daß sich, die Hakenöffnung auf der einen Seite begrenzend,vom Halteabschnitt parallel zur Achse der Durchbohrung (131) eine Lasche (133) erstreckt, die mindestens eine Bohrung (134) für eine Knochenschraube aufweist.

24. Stützvorrichtung nach Anspruch 17, dadurch gekennzeichnet, daß ein Haken (160) vorgesehen ist mit einem ringförmigen Klemmkopf (163) und dementsprechend ringförmiger Klemmfläche, die an der Klemmfläche (46) einer Pedikelschraube (40) oder eines Bolzens (10, 11; 12, 13) festklemmbar ist.

25. Stützvorrichtung nach Anspruch 17, dadurch gekennzeichnet, daß der Distraktionsstab (70, 110) seitlich von einem Haken (160) umgriffen wird, der an einem annähernd geradlinigen Halteabschnitt angebracht ist und am freien Ende des Halteabschnitts (162) seitlich auf der dem Haken gegenüberliegenden Seite eine ringförmige Klemmfläche (163) angebracht ist.

## Claims

1. Supporting device for the human spinal column comprising an elongated dorsally implantable holding means, at least two pedicle screws (40) which have a head (43) and a threaded shank (41) and the head (43) of which is mounted on the holding means pivotally about an axis which extends approximately perpendicularly to the threaded shank axis whilst the threaded shank (41) projects from the holding means, and a securing means (25, 25a) for the fixing of the pedicle screws (40) in the set angular position and at desired axial intervals from each other, characterized in that the holding means comprises at least two separate, preferably threaded bolts (10, 11; 12, 13) which cooperate with a threaded sleeve (16) and which at the head ends (16a) comprise a clamping surface (29) lying on the axis or axis-parallel, the head (43) of the pedicle screws (40) comprises two approximately parallel clamping faces (46) extending approximately parallel to the threaded shank axis and for each bolt (10, 11; 12, 13) a screw connection (35, 50) is provided which clampingly fixes with respect to each other in their position the clamping surfaces of bolts and pedicle screws or of two bolts.

2. Supporting device according to claim 1, characterized in that the clamping surfaces (29; 46) have a high roughness.

3. Supporting device according to claim 1, characterized in that the clamping surfaces (29; 46) comprise a toothing (30; 47).

4. Supporting device according to any of the claims 1 to 3, characterized in that the clamping surfaces (29) of the bolts (10, 11; 12, 13) are traversed by bores (31, 33, 34) and received by the screw bolts (35, 50).

5. Supporting device according to claim 1, characterized in that the threaded sleeve (16) comprises spanner faces (17).

6. Supporting device according to any of the claims 1 to 5, characterized by a plurality of locking means (25, 25a, 25b, 25c) cooperating with a thread of the bolts (10, 11; 12, 13) and the threaded sleeve (16) for fixing the axial and rotational position of said parts.

7. Supporting device according to claim 6, characterized in that a lock nut (25) is mounted on the thread of the bolts (10, 11; 12, 13).

8. Supporting device according to claim 6, characterized by a threaded sleeve (16) deformable by squeezing and at least one flattening on the portion of the bolt mounted in the threaded sleeve.

9. Supporting device according to claim 6, characterized in that the threaded sleeve is at least partially slit and associated to the threaded sleeve is a clamping means for fixedly clamping the threaded sleeve on at least one bolt.

10. Supporting device according to claims 6 and 7, characterized in that the bolt (10, 11) comprises at least one flattening (11a) and between the lock nut (25) and threaded sleeve (16) a washer (25a) is mounted non-rotatably on the bolt (10, 11) and comprises an end-face toothing (25b) which cooperates with an end-face toothing (25c) of the threaded sleeve (16).

11. Supporting device according to any of the claims 1 to 10, characterized in that the clamping faces (29; 46) are circular.

12. Supporting device according to any if the claims 1 to 11, characterized in that between the head (43) and the threaded shank (41) of the pedicle screws (40) a smooth cylindrical portion (44) is provided.

13. Supporting device according to any of the claims 1 to 12, characterized in that the threaded shank (41) of the pedicle screws (40) comprises a conical a core tapering towards the free end.

14. Supporting device according to any of the claims 4 to 12, characterized in that the head (36, 51) of the screw bolt (35, 50) is conical and cooperates with a conical bore portion (34, 45) of the pedicle screws (40) or bolts (10, 11), respectively.

15. Supporting device according to claim 14, characterized in that the bore portion (45) of the pedicle screws (40) is double-conical.

16. Supporting device according to claim 14 or 15, characterized in that the screw bolt head (36, 51) has sunken key faces.

17. Supporting device according to any of the claims 1 to 16, characterized in that at least one separate relatively long distraction rod (70; 110) is provided having a head (72; 112) with a clamping surface cooperating with the clamping surface (30) of the bolt (10a) as well as holding means (71; 115) at the other end for a connecting member to be attached to a vertebra.

18. Supporting device according to any of the claims 1 to 16, characterized in that the shank (63) of a bolt (60) is provided with an axial blind bore (64) for receiving a preferably cylindrical pin if a relatively long rod which at the other end is provided with holding means for a connecting member to be attached to a vertebra, the head (62) of the bolt (60) provided with the blind bore (64) being equal to the head (16a) of the bolt (10).

19. Supporting device according to any of the claims 1 to 18, characterized in that the head of the bolt comprises an insert bore for receiving a preferably cylindrical pin of a relatively long distraction rod which at one end is provided with holding means for a connecting member to be attached to a vertebra.

20. Supporting device according to any of the claims 1 to 19, characterized by an adapter (80) which at one end comprises a clamping surface (81) for a connection with a bolt and/or a pedicle screw (40) by means of the screw connection (83) as well as a fork portion (84) spaced from the clamping surface (81) for receiving an elongated, preferably threaded rod (85), the axis of the rod lying in a plane offset with the axis of the bolt and the rod (85) being lockable by means of nuts (86, 87) on the adapter (80).

21. Supporting device according to claim 17, characterized in that the connecting member is a hock (120) which comprises a holding portion (121) having a through bore through which a distraction rod (110) is led and the holding portion (121) can be secured by means of a locking screw (122) on the rod (110).

22. Supporting device according to claim 21, characterized in that the end of the hock (120) is made fork-shaped (123).

23. Supporting device according to claim 21 or 22, characterized in that a plate (133), bordering the hook opening on the one side, extends from the holding portion parallel to the axis of the through bore (131) and comprises at least one bore (134) for a bone screw.

24. Supporting device according to claim 17, characterized in that a hook (160) is provided comprising an annular clamping head (163) and a corresponding annular clamping surface for securing to the clamping surface (46) of a pedicle screw (40) or a bolt (10, 11; 12, 13).

25. Supporting device according to claim 17, characterized in that the distraction rod (70, 110) is engaged laterally by a hook (160) which is attached to an approximately rectilinear holding portion and at the free end of the holding portion (162) laterally on the side opposite the hook an annular clamping surface (163) is disposed.

## Revendications

1. Dispositif d'étaiement de la colonne vertébrale humaine, constitué d'un dispositif de retenue allongé, pouvant être implanté dans le dos, d'au moins deux vis pédiculaires (40) présentant une tête (43) et une tige filetée (41), dont la tête (43) est montée sur le dispositif de retenue, de façon à pouvoir pivoter autour d'un axe qui s'étend sensiblement perpendiculairement par rapport à l'axe de la tige filetée, alors que la tige filetée (41) s'écarte du dispositif de retenue, et d'un dispositif de fixation (25, 25a) pour maintenir les vis pédiculaires (40) dans la position angulaire réglée et selon des écartements axiaux souhaités les unes par rapport aux autres,
caractérisé en ce que le dispositif de retenue présente au moins deux goujons (10, 11; 12, 13) séparés, de préférence filetés, qui coopèrent avec une douille (16) taraudée, et qui présentent aux extrémités de tête (16a) une surface de serrage (29) située sur l'axe ou parallèle à l'axe, la tête (43) des vis pédiculaires (40) présentant deux surfaces de serrage (46) sensiblement parallèles, s'étendant sensiblement parallèlement à l'axe de la tige filetée, et en ce qu'un assemblage vissé (35, 50), prévu pour chaque goujon (10, 11 ; 12, 13), maintient dans leur position, en les serrant l'une contre l'autre, les surfaces de serrage des goujons et des vis pédiculaires ou de deux goujons.

2. Dispositif d'étaiement selon la revendication 1, caractérisé en ce que les surfaces de serrage (29, 46) sont très rugueuses.

3. Dispositif d'étaiement selon la revendication 1, caractérisé en ce que les surfaces de serrage (29, 46) présentent une denture (30 ; 47).

4. Dispositif d'étaiement selon l'une quelconque des revendications 1 à 3, caractérisé en ce que les surfaces de serrage (29) des goujons (10, 11 ; 12, 13) sont percées de trous (31, 33, 34) et sont en prise avec des tiges filetées (35, 50).

5. Dispositif d'étaiement selon la revendication 1, caractérisé en ce que la douille taraudée (16) présente des surfaces pour clé (17).

6. Dispositif d'étaiement selon l'une quelconque des revendications 1 à 5, caractérisé en ce que le dispositif de fixation présente plusieurs moyens de fixation (25, 25a, 25b, 25c) coopérant avec un filetage des goujons (10, 11, 12, 13) et de la douille taraudée, pour maintenir la position axiale et de rotation de ces pièces.

7. Dispositif d'étaiement selon la revendication 6, caractérisé en ce qu'un contre-écrou (25) est en prise avec le filetage des goujons (10, 11, 12, 13).

8. Dispositif d'étaiement selon la revendication 6, caractérisé par une douille filetée (16) pouvant être déformée par écrasement et au moins un aplatissement sur la section du goujon pénétrant dans la douille filetée.

9. Dispositif d'étaiement selon la revendication 6, caractérisé en ce que la douille filetée est au moins en partie pourvue de fentes et qu'un dispositif de serrage est associé à la douille filetée, pour bloquer la douille filetée sur au moins un goujon.

10. Dispositif d'étaiement selon l'une quelconque des revendications 6 et 7, caractérisé en ce que le goujon (10, 11) présente au moins un aplatissement (11a), en ce qu'une rondelle (25a) est montée sur le goujon (10, 11), sans rotation possible, entre le contre-écrou (25) et la douille filetée (16), ladite rondelle présentant en face frontale une denture (25b) qui coopère avec une denture (25c) de la face frontale de la douille filetée (16).

11. Dispositif d'étaiement selon l'une quelconque des revendications 1 à 10, caractérisé en ce que les surfaces de serrage (29; 46) sont de forme circulaire.

12. Dispositif d'étaiement selon l'une quelconque des revendications 1 à 11, caractérisé en ce qu'une partie (44) cylindrique lisse est prévue entre la tête (43) et la tige filetée (41) des vis pédiculaires (40).

13. Dispositif d'étaiement selon l'une quelconque des revendications 1 à 12, caractérisé en ce que la tige filetée (41) des vis pédiculaires (40) présente une âme conique rétrécissant vers l'extrémité libre.

14. Dispositif d'étaiement selon l'une quelconque des revendications 4 à 12, caractérisé en ce que la tête (36, 51) de la tige filetée (35, 50) est conique et coopère avec une section de trou conique (34, 45) des vis pédiculaires (40) ou des goujons (10, 11).

15. Dispositif d'étaiement selon la revendication 14, caractérisé en ce que la section de trou (45) des vis pédiculaires (40) est en forme de cône double.

16. Dispositif d'étaiement selon l'une quelconque des revendications 14 ou 15, caractérisé en ce que la tête du goujon fileté (36, 51) présente des surfaces obliques pour clé.

17. Dispositif d'étaiement selon l'une quelconque des revendications 1 à 16, caractérisé en ce qu'au moins une barre d'extension allongée (70 ; 110), séparée est prévue, comportant une tête (72 ; 112) présentant une surface de serrage, qui coopère avec la surface de serrage (30) du goujon (10a), ainsi qu'avec des moyens de retenue (71 ; 115) à l'autre extrémité, pour un élément de raccord devant être apposé sur une vertèbre.

18. Dispositif d'étaiement selon l'une quelconque des revendications 1 à 16, caractérisé en ce que la tige (63) d'un goujon (60) est pourvue d'un trou borgne (64) pour loger un pivot, de préférence cylindrique qui fait partie d'une barre allongée, qui est pourvue à l'autre extrémité de moyens de retenue, pour un élément de raccord devant être apposé sur une vertèbre, la tête (62) du goujon (60) comportant le trou borgne (64) étant identique à la tête (16a) du goujon (10).

19. Dispositif d'étaiement selon l'une quelconque des revendications 1 à 18, caractérisé en ce que la tête du goujon comporte un trou d'insertion, pour loger un pivot de préférence cylindrique qui fait partie d'une barre d'extension allongée, qui est pourvue, à une extrémité, de moyens de retenue, pour un élément de raccordement devant être apposé sur une vertèbre.

20. Dispositif d'étaiement selon l'une quelconque des revendications 1 à 19, caractérisé par un adaptateur (80) qui présente à une extrémité une surface de serrage (81) en vue de l'assemblage avec un goujon et/ou une vis pédiculaire (40), au moyen d'un assemblage vissé (83) ainsi qu'une section formant fourche (84) à distance de la surface de serrage (81) pour loger une barre (85) allongée, de préférence filetée, l'axe de la barre étant situé dans un plan décalé par rapport au plan traversant l'axe du goujon, et la barre (85) pouvant être fixée au moyen d'écrous (86, 87) sur l'adaptateur (80).

21. Dispositif d'étaiement selon la revendication 17, caractérisé en ce que l'élément de raccord est un crochet (120), qui présente une section de retenue (121) avec un trou traversant, par lequel est insérée une barre d'extension (110), et que la section de retenue (121) peut être immobilisée au moyen d'une vis de fixation (122) sur la barre (110).

22. Dispositif d'étaiement selon la revendication 21, caractérisé en ce que l'extrémité du crochet (120) est conformée comme une fourche (123).

23. Dispositif d'étaiement selon l'une quelconque des revendications 21 ou 22, caractérisé en ce qu'une patte (133), qui présente au moins un trou (134) prévu pour une vis pour os, s'étend à partir de la section de retenue, parallèlement à l'axe du trou traversant (131), en limitant, d'un côté, l'ouverture du crochet.

24. Dispositif d'étaiement selon la revendication 17, caractérisé en ce qu'un crochet (160) est prévu avec une tête de serrage (163) de forme annulaire et une surface de serrage de forme annulaire correspondante, qui peut être fixée sur la surface de serrage (46) d'une vis pédiculaire (40) ou d'un goujon (10, 11; 12, 13).

25. Dispositif d'étaiement selon la revendication 17, caractérisé en ce que la barre d'extension (70, 110) est enserrée latéralement par un crochet (160), qui est monté sur une section de retenue sensiblement rectiligne et en ce qu'une une surface de serrage annulaire (163) est disposée à l'extrémité libre de la section de retenue (162), latéralement, sur le côté opposé au crochet.
